# EUROPEAN PATENT SPECIFICATION

(11) **EP 3 341 495 B1**
(45) Date of publication and mention of the grant of the patent: **07.06.2023**
(21) Application number: 16763978.0
(22) Date of filing: 25.08.2016
(51) Int. Cl.: C12Q 1/68

(54) **ZIC1 AND GHSR, MOLECULAR DIAGNOSTIC MARKERS FOR HPV-INDUCED INVASIVE CANCERS, NONHPV-INDUCED GYNAECOLOGICAL AND ANOGENITAL CANCERS AND THEIR HIGH-GRADE PRECURSOR LESIONS**
ZIC1 UND GHSR, MOLEKULARE DIAGNOSEMARKER FÜR HPV-INDUZIERTE INVASIVE KREBSERKRANKUNGEN, NICHT-HPV-INDUZIERTE GYNÄKOLOGISCHE UND ANOGENITALE KREBSERKRANKUNGEN UND DEREN HOCHGRADIGE VORLÄUFERVERLETZUNGEN
ZIC1 ET GHSR, MARQUEURS DE DIAGNOSTIC MOLÉCULAIRE POUR CANCERS INVASIFS CAUSÉS PAR LE VPH, CANCERS GYNÉCOLOGIQUES ET ANOGÉNITAUX NON CAUSÉS PAR LE VPH ET LEURS LÉSIONS PRÉCANCÉREUSES DE HAUT GRADE

(30) Priority: 26.08.2015 EP 15182601
(43) Date of publication of application: 04.07.2018
(73) Proprietor: Self-screen B.V., 1098 RX Amsterdam (NL)
(72) Inventor: HESSELINK, Albertus Theodorus, 1098 RX Amsterdam (NL)
(74) Representative: V.O.
(86) International application number: PCT/NL2016/050593
(87) International publication number: WO 2017/034407

(56) References cited:
- WO-A1-2013/039394
- WO-A1-2014/058321
- WO-A1-2014/086374
- WO-A1-2016/115354
- JP-A- 2011 160 711
- XUEQIN CHEN ET AL: "Zic1 Promoter Hypermethylation in Plasma DNA Is a Potential Biomarker for Gastric Cancer and Intraepithelial Neoplasia", PLOS ONE, vol. 10, no. 7, 24 July 2015 (2015-07-24), page e0133906, XP055252361, DOI: 10.1371/journal.pone.0133906
- WANG L J ET AL: "ZIC1 is downregulated through promoter hypermethylation in gastric cancer", BIOCHEMICAL AND BIOPHYSICAL RESEARCH COMMUNICATIONS, ACADEMIC PRESS INC. ORLANDO, FL, US, vol. 379, no. 4, 20 February 2009 (2009-02-20), pages 959-963, XP025928722, ISSN: 0006-291X, DOI: 10.1016/J.BBRC.2008.12.180 [retrieved on 2009-01-09]
- WEI QIANG ET AL: "ZIC1 Is a Putative Tumor Suppressor in Thyroid Cancer by Modulating Major Signaling Pathways and Transcription Factor FOXO3a", JOURNAL OF CLINICAL ENDOCRINOLOGY AND METABOLISM, vol. 99, no. 7, 1 July 2014 (2014-07-01), pages E1163-E1172, XP055252387, US ISSN: 0021-972X, DOI: 10.1210/jc.2013-3729
- POURIA JANDAGHI ET AL: "GHSR hypermethylation: a promising pan-cancer marker", CELL CYCLE, vol. 14, no. 5, 20 January 2015 (2015-01-20), pages 689-690, XP055252375, US ISSN: 1538-4101, DOI: 10.1080/15384101.2015.1006051
- BOTLA SANDEEP KUMAR ET AL: "Diagnostic values of GHSR DNA methylation pattern in breast cancer", BREAST CANCER RESEARCH AND TREATMENT, vol. 135, no. 3, October 2012 (2012-10), pages 705-713, XP002754649,
- Rui-Lan Huang ET AL: "Comprehensive methylome analysis of ovarian tumors reveals hedgehog signaling pathway regulators as prognostic DNA methylation biomarkers", EPIGENETICS, vol. 8, no. 6, 1 June 2013 (2013-06-01), pages 624-634, XP055558834, US ISSN: 1559-2294, DOI: 10.4161/epi.24816
- Lihong Gan ET AL: "ZIC1 Is Downregulated through Promoter Hypermethylation, and Functions as a Tumor Suppressor Gene in Colorectal Cancer", PLoS ONE, vol. 6, no. 2, 15 February 2011 (2011-02-15), page e16916, XP055558915, DOI: 10.1371/journal.pone.0016916

## Description

### FIELD OF THE INVENTION

The invention relates to the field of cancer prevention and medical diagnostics; and is concerned with a molecular diagnostic marker for cancers, especially human papillomavirus (HPV)-induced invasive cancers and high-grade precursor lesions thereof, such as invasive cervical cancer and premalignant cervical lesions, nonHPV-induced gynaecological and anogenital cancers. In particular, the present invention relates to the use of the ZIC 1 and GHSR genomic and regulatory sequence as marker for hrHPV-induced invasive cancers, nonHPV-induced gynaecological and anogenital cancers and their premalignant lesions with invasive potential.

### BACKGROUND OF THE INVENTION

Cancer of the uterine cervix is the fourth most common cancer in women world-wide and is responsible for approximately 250.000 cancer deaths a year.

Cervical squamous cell carcinoma development is characterized by a sequence of premalignant lesions, so-called cervical intraepithelial neoplasia (CIN), which are graded 1 to 3, referring to mild dysplasia (CIN 1), moderate dysplasia (CIN 2) and severe dysplasia/carcinoma *in situ* (CIN 3), respectively. CIN 1 is also referred to as low grade squamous intraepithelial lesion (LSIL) and CIN 2 and CIN 3 together as high grade squamous intraepithelial lesion (HSIL). For cervical adenocarcinoma, adenocarcinoma in situ (ACIS) is an established precursor lesion. In principle, these premalignant lesions are reversible, although the more severe the lesion, the lower the chance of spontaneous regression. Cervical cancer is considered a preventable disease because the premalignant stages can be detected by exfoliative cytology and treated relatively easily when necessary, with only minor side effects. Cervical screening is aimed to early diagnose the high-grade premalignant (i.e., CIN 2/3 and adenocarcinoma in situ) and treatable cancerous lesions, thereby reducing the mortality of invasive cervical cancer. General medical practice comprises the treatment of all women with morphologically confirmed CIN 2, CIN 3 and adenocarcinoma in situ, in order to prevent the development of cervical cancer.

Over the past decade it has been well established that cervical carcinogenesis is initiated by an infection with high-risk human papillomavirus (hrHPV). Expression of the viral oncogenes E6 and E7, which disturb the p53 and Rb tumor suppressor pathways, respectively, has been shown to be essential for both the onset of oncogenesis and the maintenance of a malignant phenotype. Therefore, testing for hrHPV appeared as an attractive, primary screening tool. However, consistent with a multistep process of carcinogenesis, additional alterations in the host cell genome are required for progression of an hrHPV infected cell to invasive cancer cell. Only a small proportion of women infected with high-risk HPV will develop high-grade premalignant cervical lesions (CIN 2/3) and, if left untreated, cervical cancer. In most women with premalignant cervical lesions the lesions regress spontaneously. Of the women who participate in population based screening, about 5-6% have a positive hrHPV test. However, only at maximum 20% of them (1% of the participating women) have ≥CIN 2/3. Therefore, primary screening by hrHPV testing will be accompanied with a substantial number of redundant follow-up procedures and unnecessary anxiety amongst women, unless markers can be applied to the cervical smears that allow stratification of hrHPV positive women for risk of ≥CIN 2/3 and ≥adenocarcinoma in situ.

A major challenge is to reduce the percentage of HPV test positive women to those that have clinically meaningful lesions. One mode is to use cytology as a secondary (so-called triage) test for hrHPV positive women. Still, this leaves a substantial number of hrHPV positive women with normal cytology (3.5% of the women in the screening population), of which still 10% have or acquire ≥CIN 3. Moreover, cytology is not an option for self-sampled cervico-vaginal specimens that can be taken at home, since these are not representative for the cytological status of the cervix. Another mode is to use HPV16/18 genotyping. This however leaves women with nonHPV16/18 types who are, although to a lesser extent, also at risk of ≥CIN 2/3 and ≥adenocarcinoma in situ. Therefore, there is a need for supplementary or alternative triage tools to stratify hrHPV positive women into those with and without ≥CIN 2/3 and ≥adenocarcinoma in situ.

Primary screening for cervical cancer using disease markers based on host cell changes in cancer genes provides a promising alternative provided that specificity and sensitivity is sufficiently high. This option is of particular interest for low and middle income countries, where qualitycontrolled cytology is absent and implementation of follow-up algorithms for HPV-positive women is complicated. In these countries self-sampling has shown to facilitate access to cervical screening (Laczano-Ponce et al., Lancet. 2011; 378: 1868-1873). In this sense it is extremely useful to have markers that also prevail in self-samples. It appears that there is a huge difference in the 'behaviour' of markers on vaginal smears that have been obtained by medical skilled personnel like doctors or nurses and on vaginal swabs that have been collected by the woman herself. It has appeared that many markers that would be suitable for doctor-provided samples are not useful in self-samples. The necessity to work with self-samples in stead of doctor samples is high in low and middle income countries since in those countries there is less medical personnel per capita and often there is a cultural problem by letting other persons taking a vaginal sample.

Endometrial cancer is the most common gynaecologic malignancy in many developed countries (Siegel et al., CA Cancer J. Clin., 64 (2014), pp. 9-29). Early stage endometrial cancer has a very good prognosis. Therefore, early detection will increase the chance of cure and avoid or reduce cumbersome and costly therapeutic intervention. As shedding of tumor cells in cervical scrapes has been demonstrated, early detection of endometrial cancer by non-invasive sampling is feasible. However, conventional cytology on cervical scrapes has a very low sensitivity for detection of endometrial cancer. Testing for molecular alterations, such as DNA methylation, associated with endometrial cancer, may provide a promising approach for early detection of endometrial cancer (de Strooper et al., J Clin Pathol. 2014;67(12):1067-71, Bakkum-Gamez et al. Gynecol Oncol. 2015;137(1):14-22). Similarly, testing for cancer specific DNA alterations in cervical scrapes for the detection of ovarian cancer has recently been proposed (Kindle et al., Sci Transl Med. 2013; 5(167):1-21).

### SUMMARY OF THE INVENTION

The inventors now have found a method for detecting HPV-induced invasive cancers, nonHPV-induced gynaecological and anogenital cancers, and their high-grade precancerous lesions wherein said method comprises the detection of hypermethylation in the ZIC 1 and/or GHSR gene in a cell whereby such hypermethylation indicates the presence of HPV-induced precursor lesions with invasive potential, HPV-induced invasive cancers, nonHPV-induced gynaecological and anogenital cancers. Preferably, in such a method said HPV-induced high-grade precancerous lesion or HPV-induced invasive carcinoma is a high-grade premalignant cervical lesion or invasive cervical cancer, more preferably a high-risk HPV-induced invasive cancer. Said nonHPV-induced gynaecological cancer is a endometrial cancer. Said nonHPV-induced anogenital cancer is a vulvar cancer.

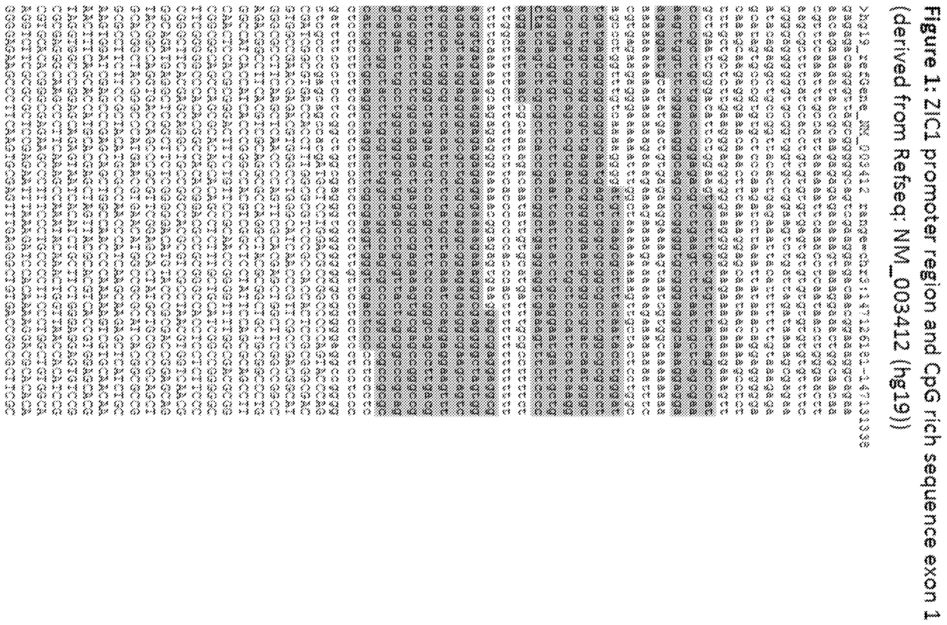

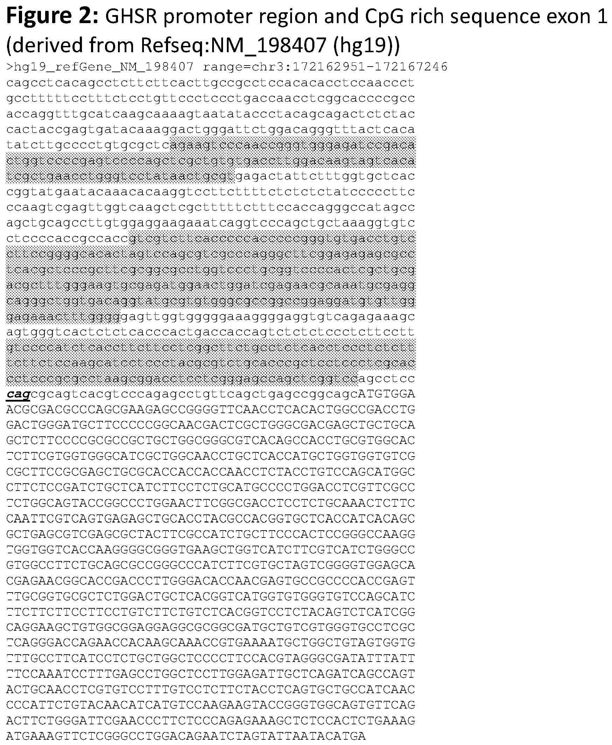

In a preferred embodiment of the invention the hypermethylation is detected in the CpG rich sequences as indicated in Figures 1 and 2.

The invention also relates to a method as defined above wherein said hypermethylation is an increased methylation of ZIC1 and/or GHSR CpG rich promoter and /or genomic sequences in the test cell as compared to the comparable normal cell.

In a preferred embodiment of the invention the detection of (hyper)methylation is performed by using a methylation sensitive restriction endonuclease, chosen from the group consisting of BssHII, MspI, NotI and HpaII. In another preferred embodiment of the invention the detection of (hyper)methylation is performed using nanotechnology. In an alternative preferred embodiment of the invention, the detection of (hyper)methylation is performed via a methylation specific PCR, which is based on bisulfite modification of DNA, followed by specific PCR reactions that target CpG rich sequences. Preferably in such a method the reagent is a nucleic acid probe or primer that binds to the nucleic acid as indicated in Figure 1 or 2, and more preferably said nucleic acid probe or primer has a detectable label.

In another embodiment of the invention the nucleic acid probe has a nucleotide sequence selected from the group consisting of:
a) a polynucleotide sequence capable of hybridizing under stringent conditions to the sequence ZIC1 as set forth in Figure 1 or to the sequence GHSR as set forth in Figure 2;
b) a polynucleotide having at least 70% identity to the polynucleotide of a);
c) a polynucleotide complementary to the polynucleotide of a); and
d) a polynucleotide comprising at least 15 bases of a nucleotide of a) of b).

Further preferred in the present method of the invention the methylation of both the ZIC1 and the GHSR gene is determined.

Also part of the invention is the use of ZIC1 and/or GHSR as a molecular diagnostic marker for the detection of HPV-induced invasive carcinoma, nonHPV-induced gynaecological or anogenital carcinoma, and their high-grade precancerous lesion,. Preferably, in such a use the methylation of said marker is predictive for the occurrence of said lesion or carcinoma.

The invention also comprises a kit of parts for use in a method of detecting HPV-induced invasive carcinoma, nonHPV-induced gynaecological or anogenital carcinoma and their high- grade precancerous lesion said kit comprising
- means for the detection of ZIC 1 and/or GHSR methylation wherein said means comprise probes and/or primers specific for the ZIC1 nucleotide sequence of Figure 1 and/or the GHSR nucleotide sequence of Figure 2 and
- means for the detection of HPV infection, wherein said means comprise probes and primers specific for HPV.

### BRIEF DESCRIPTION OF THE DRAWINGS

Figure 1 shows the *ZIC1* 5' regulatory region and coding sequence. Transcription start is indicated in bold italic underlined, CpG islands shaded in grey and coding sequence is in upper case.
Figure 2 shows the *GHSR* 5' regulatory region and coding sequence. Transcription start is indicated in bold italic underlined, CpG islands shaded in grey and coding sequence is in upper case.

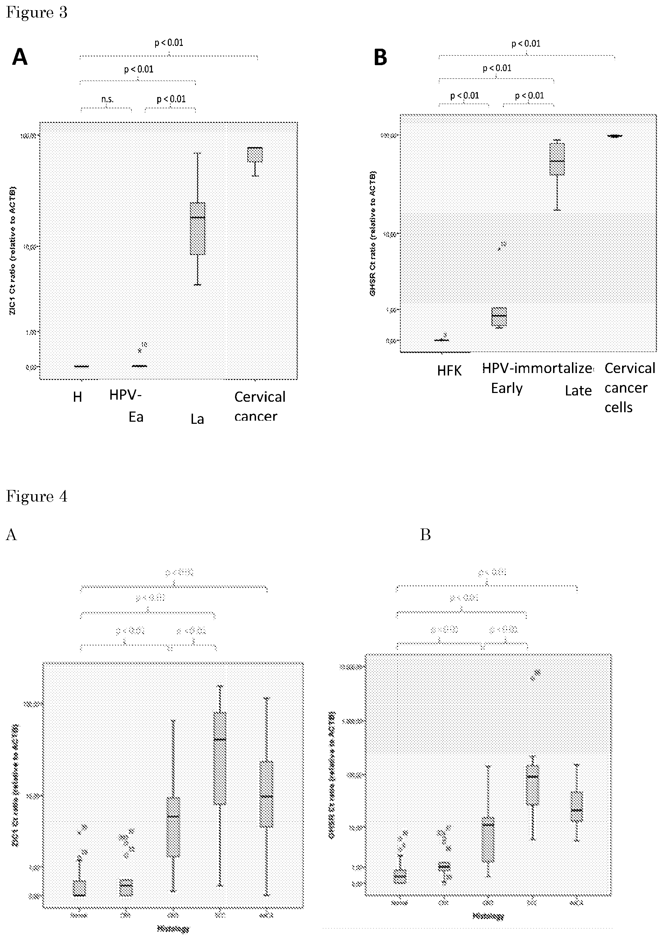

Figure 3 shows boxplots of the levels of ZIC 1 and GHSR as measured by quantitative methylation specific PCR in primary keratinocytes, hrHPV-immortalized keratinocytes and cervical cancer cell lines. On the y-axes levels of methylated DNA are presented; on the x-axes the cell lines are grouped according to degree of transformation (primary keratinocytes, early passage HPV16 and HPV18 immortalized keratinocytes, late passage HPV16 and HPV18 immortalized keratinocytes and cervical cancer cells). The level of methylation of ZIC1 and GHSR increases with stage of transformation and is significantly increased in late passage HPV16 and HPV18 immortalized keratinocytes and cervical cancer cells compared to primary keratinocytes and early passage HPV16 and HPV18 immortalized keratinocytes. Significant differences in methylation levels between cell categories are indicated.
Figure 4 shows boxplots of the levels of ZIC 1 and GHSR as measured by quantitative methylation specific PCR in cervical tissue specimens. On the y-axes levels of methylated DNA are presented; on the x-axes the samples are grouped for each disease stage and histotype. The level of methylation of ZIC1 and GHSR increases with stage of disease and is detected in (virtually) all SCC and AdCA. Significant differences in methylation levels between disease categories are indicated.

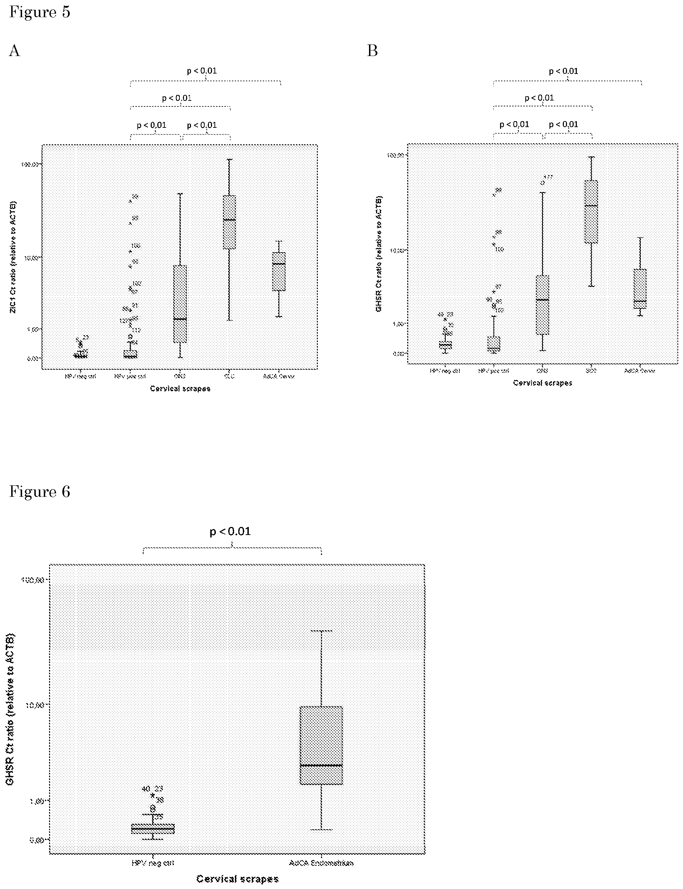

Figure 5 shows boxplots of the levels of ZIC 1 and GHSR as measured by quantitative methylation specific PCR in cervical scrapes of women without cervical disease (including both HPV negative and HPV positive scrapes), women with CIN3, women with SCC and women with AdCA. On the y-axes levels of methylated DNA are presented; on the x-axes the samples are grouped for each disease stage and histotype. The level of methylation of ZIC1 and GHSR increases with stage of disease and is detected in all SCC and AdCA. Significant differences in methylation levels between disease categories are indicated.
Figure 6 shows boxplots of the levels of GHSR as measured by quantitative methylation specific PCR in cervical scrapes of women with endometrial carcinomas. On the y-axes levels of methylated DNA are presented; on the x-axes the samples are grouped as scrapes of women without cervical disease and scrapes of women with endometrial cancer. GHSR methylation levels are significantly increased in virtually all women with endometrial carcinoma compared to controls.

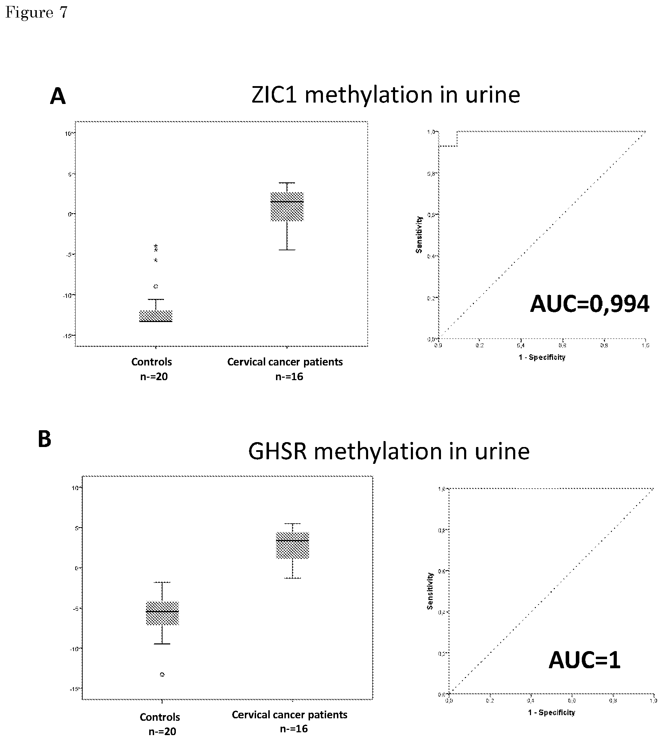

Fig. 7 shows boxplots and ROC curves of the levels of ZIC1 (A) and GHSR (B) as measured by quantitative methylation specific PCR in urine samples of women with cervical cancer. Left panel: On the y-axes levels of methylated DNA are presented; on the x-axes the samples are grouped as urine of control women (n=20) and urine of women with cervical cancer (n=16). ZIC1 and GHSR methylation levels are significantly increased in women with cervical cancer compared to controls. ROC curves in the right panels show an excellent distinction between cancer patients and controls, with AUC of 0.994 for ZIC1 and an AUC of 1,0 for GHSR.

### DETAILED DESCRIPTION OF THE INVENTION

The term "HPV-induced invasive cancer" refers to a carcinoma induced by high-risk HPV, which invades surrounding tissue. This includes all HPV-induced carcinoma histotypes, i.e., squamous cell carcinomas, adenocarcinomas, adenosquamous carcinomas and neuroendocrine carcinomas in relevant organs such as cervix, oral cavity, oropharynx, anus, rectum, penis, vulva, vagina, etc. It especially includes head and neck squamous cell carcinomas (HNSCC), cervical squamous cell carcinomas and cervical adenocarcinomas.

The term "invasive cervical cancer" refers to a cervical carcinoma invading surrounding tissue. This includes all carcinoma histotypes, i.e., squamous cell carcinomas, adenocarcinomas, adenosquamous cell carcinomas and neuroendocrine carcinomas.

The term "nonHPV-induced gynaecological or anogenital cancer" refers to endometrial cancer, ovarian cancer, vulvar cancer, vaginal cancer, anal cancer and penile cancer that are HPV-negative.

The terms "premalignant lesion" and "precursor lesion" refer to a stage in the multistep cellular evolution to cancer with a strongly increased chance to progress to a carcinoma. With classical morphology the pathologist is unable to predict in the individual patient which of these lesions will progress or regress. The current patent refers to a method, which can predict invasive cancer or a high-grade precursor lesion thereof.

The term "high-grade premalignant cervical lesion" refers to a stage in the multistep cellular evolution to cervical cancer with a strongly increased chance to progress to a cervical carcinoma. The term "capable of specifically hybridizing to" refers to a nucleic acid sequence capable of specific base-pairing with a complementary nucleic acid sequence and binding thereto to form a nucleic acid duplex.

A "complement" or "complementary sequence" is a sequence of nucleotides which forms a hydrogen-bonded duplex with another sequence of nucleotides according to Watson-Crick base-paring rules. For example, the complementary base sequence for 5'-AAGGCT-3' is 3'-TTCCGA-5'.

The term "stringent hybridization conditions" refers to hybridization conditions that affect the stability of hybrids, e.g., temperature, salt concentration, pH, formamide concentration and the like. These conditions are empirically optimised to maximize specific binding and minimize non-specific binding of the primer or the probe to its target nucleic acid sequence. The terms as used include reference to conditions under which a probe or primer will hybridise to its target sequence, to a detectably greater degree than other sequences (e.g. at least 2-fold over background). Stringent conditions are sequence dependent and will be different in different circumstances. Longer sequences hybridise specifically at higher temperatures. Generally, stringent conditions are selected to be about 5°C lower than the thermal melting point (Tₘ) for the specific sequence at a defined ionic strength and pH. The Tₘ is the temperature (under defined ionic strength and pH) at which 50% of a complementary target sequence hybridises to a perfectly matched probe or primer. Typically, stringent conditions will be those in which the salt concentration is less than about 1.0 M Na ion, typically about 0.01 to 1.0 M Na ion concentration (or other salts) at pH 7.0 to 8.3 and the temperature is at least about 30°C for short probes or primers (e.g. 10 to 50 nucleotides) and at least about 60°C for long probes or primers (e.g. greater than 50 nucleotides). Stringent conditions may also be achieved with the addition of destabilizing agents such as formamide. Exemplary low stringent conditions or "conditions of reduced stringency" include hybridization with a buffer solution of 30% formamide, 1 M NaCl, 1% SDS at 37°C and a wash in 2x SSC at 40°C. Exemplary high stringency conditions include hybridization in 50% formamide, 1 M NaCl, 1% SDS at 37°C, and a wash in 0.1x SSC at 60°C. Hybridization procedures are well known in the art and are described in e.g. Ausubel et al, Current Protocols in Molecular Biology, John Wiley & Sons Inc., 1994.

The term "oligonucleotide" refers to a short sequence of nucleotide monomers (usually 6 to 100 nucleotides) joined by phosphorous linkages (e.g., phosphodiester, alkyl and aryl-phosphate, phosphorothioate), or nonphosphorous linkages (e.g., peptide, sulfamate and others). An oligonucleotide may contain modified nucleotides having modified bases (e.g., 5-methyl cytosine) and modified sugar groups (e.g., 2'-O-methyl ribosyl, 2'-O-methoxyethyl ribosyl, 2'-fluoro ribosyl, 2'-amino ribosyl, and the like). Oligonucleotides may be naturally-occurring or synthetic molecules of double- and single-stranded DNA and double- and single-stranded RNA with circular, branched or linear shapes and optionally including domains capable of forming stable secondary structures (e.g., stem-and-loop and loopstem-loop structures).

The term "primer" as used herein refers to an oligonucleotide which is capable of annealing to the amplification target allowing a DNA polymerase to attach thereby serving as a point of initiation of DNA synthesis when placed under conditions in which synthesis of primer extension product which is complementary to a nucleic acid strand is induced, i.e., in the presence of nucleotides and an agent for polymerization such as DNA polymerase and at a suitable temperature and pH. The (amplification) primer is preferably single stranded for maximum efficiency in amplification. Preferably, the primer is an oligodeoxy ribonucleotide. The primer must be sufficiently long to prime the synthesis of extension products in the presence of the agent for polymerization. The exact lengths of the primers will depend on many factors, including temperature and source of primer. A "pair of bi-directional primers" as used herein refers to one forward and one reverse primer as commonly used in the art of DNA amplification such as in polymerase chain reaction (PCR) amplification.

The term "probe" refers to a single-stranded oligonucleotide sequence that will recognize and form a hydrogen-bonded duplex with a complementary sequence in a target nucleic acid sequence analyte or its cDNA derivative.

DNA methylation is a biochemical process that is important for normal development in higher organisms. It involves the addition of a methyl group to the 5 position of the cytosine pyrimidine ring or the number 6 nitrogen of the adenine purine ring. DNA methylation at the 5 position of cytosine has the specific effect of reducing gene expression and has been found in every vertebrate examined. In adult somatic tissues, DNA methylation typically occurs in a CpG dinucleotide context.

Using a genome wide DNA methylation screen on cervical tissue specimens and HPV-immortalized cell lines it has now been found that the gene encoding Zic family member 1 (further referred to as *ZIC1*; Genbank Accession NM_003412) and the gene encoding Growth Hormone Secretagogue Receptor (further referred to as *GHSR*; Genbank Accession NM_198407) are targeted by DNA methylation in primary keratinocytes immortalized by full length HPV16 and HPV18 as well as in CIN3 lesions and cervical carcinomas, and that *ZIC1* and *GHSR* promoter methylation are important determinants of hr-HPV induced carcinogenesis. The *ZIC1* and *GHSR* genomic and regulatory sequences thus provide valuable markers to diagnose invasive cervical cancer and the high-grade precursor lesions thereof. Additionally, the present invention is suited to diagnose non-cervical hrHPV-associated invasive cancers and their high-grade precursor lesions. Moreover, GHSR methylation is additionally suited to diagnose endometrial and other nonHPV- induced gynaecological and anogenital cancers.

Cervical cancer is almost exclusively associated with human papillomavirus (HPV) infection. Human papillomaviruses, constitute a group of more than 150 types of viruses, as identified by variations in DNA sequence. The various HPVs cause a variety of cutaneous and mucosal diseases. HPVs are broadly classified into low-risk and high-risk types, based on their ability to induce malignant changes in infected cells. Low risk HPV types such as 1, 2, 4, 6, 11, 13 and 32 are primarily associated with benign lesions or common warts, while the high risk types, such as 16, 18, 31, 33, 35, 39, 45, 51, 52, 56, 58, 59, 66, and 68 are primarily associated with premalignant and malignant epithelial lesions. The high-risk HPV types have been found to cause invasive carcinoma of the uterine cervix, as well as invasive carcinoma elsewhere in the anogenital tract and/or headneck region. Therefore, the present invention is not only suited to detect invasive cervical cancer and precursor stages thereof, but also other invasive cancers and corresponding precursor stages that are induced by HPV, particularly of the high-risk type. Thus, the present invention provides a method for the risk assessment of any HPV-induced high-grade premalignant lesion or invasive cancer.

Very suitable HPV-induced precursor lesions and invasive cancers in the context of the present invention are cervical precancerous lesions and invasive cervical cancers, but also precursor lesions and invasive cancers induced by high-risk HPV in other tissues such as oral cavity, oropharynx, anus, rectum, penis, vulva, vagina, etc.

As stated above, the GHSR marker also is capable of detecting nonHPV-induced precursor lesions and invasive cancers. In the context of the present invention such cancers are endometrial cancer, and vulvar cancer that are HPV-negative .

A test cell may be a (pre)neoplastic cell, a proliferating cervical cell, or any other cell wherein the presence of an HPV-induced precursor lesion with invasive potential, HPV-induced invasive cancer, nonHPV-induced gynaecological and anogenital cancer is to be detected.

The ZIC1 gene encodes a 48 kDa protein that functions as a transcription factor and is a member of the ZIC family of C2H2-type zinc finger proteins. Members of this family are important during development. ZIC1 is involved in neurogenesis. It plays important roles in the early stage of organogenesis of the CNS, as well as during dorsal spinal cord development and maturation of the cerebellum (reviewed by Grinberg and Millen, Clin Genet. 2005, 67(4):290-6). ZIC1 hypermethylation has been described in colorectal, gastric, ovarian and hepatocellular cancer (Gan et al., PLoS One. 2011, 6(2):e16916; Wang et al., Biochem Biophys Res Commun. 2009,379(4):959-63; Huang et al., Epigenetics. 2013, 8(6):624-34; Wang et al., Tumour Biol. 2014,35(8):7429-33). It has been mentioned as one of the factors that was found in a screen of hypermethylated genes in carcinoma *in situ* and cancer in a cervical swab (Wang et al., Cancer Med. 2015, 4(1):43-55) but only as one out of more than 2200 genes.

The *GHSR* gene, encodes a 41kDa protein that is a member of the G-protein coupled receptor family. The encoded protein may play a role in energy homeostasis and regulation of body weight (Howard et al., Science 1996,273(5277):974-7). GHSR hypermethylation has been described in cancers of the lung, breast, prostate, pancreas, colorectum, glioblastoma and B cell chronic lymphocytic leukemia (Moskalev et al., Oncotarget 2015,6(6):4418-27). Although it has been characterized as a pan-cancer marker, no link with HPV-induced cancers has yet been disclosed. It is surprising that this marker, that was found in other cancer types also appears to be a marker for HPV-induced cancer. It has been found in studies of head and neck squamous carcinoma (HNSCC), which are often caused by hrHPVs, that there is a difference in the methylome of HNSCCs that are HPV-induced and those that are not related to HPV (Sartor et al., 2011, Epigenetics 6(6):777-787). This was endorsed in other studies, where e.g. Colacino et al. (2013 PLOS One, 8(1):E54742 showed that hypermethylation of SPDEF, RASSF1, STAT5A, MGMT, ESR2, JAK3 and HSD17B12 is significantly associated with HPV-negative HNSCC. Similarly, Kostarelli et al. (J.Clin Investigation, 2013,123(6): 2488-2501) showed methylation of ALDH1A2, FKBP4, GDNF, OSR2, PROX1 and WIF1 to be significantly increased in HPV-negative HNSCC compared to normal mucosa, whereas these genes showed no hypermethylation in HPV-induced HNSCC. CDKN2A methylation as studied in oropharyngeal cancers was exclusively detected in HPV-negative cancers and absent in HPV-positive cancers (Taioli et al., 2009, BMC Cancer, 9: e354). Also in vulvar cancers methylations patterns differ between HPV-induced cancers and those not related to HPV, e.g. vulvar cancers associated with lichen sclerosus (LS) (Guerrero et al., 2010, Int J Cancer 28:2853-2864). For example, RASSF2A was found to be exclusively methylated in vulvar cancers associated with LS, that are HPV-negative. No RASSF2A methylation was detected in HPV-induced vulvar cancers.

The present inventors have now established that *ZIC1* and *GHSR* promoter methylation is a frequent event in cervical carcinomas of both squamous cell carcinoma, adeno-squamous carcinoma, adenocarcinoma and neuroendocrine carcinoma histotypes, and their high-grade precursor lesions. Most interestingly, the present inventors have shown that hypermethylation of the ZIC1 and GHSR promoter can be detected in cervical scrape samples and that this feature is able to predict the presence of a high-grade CIN lesion or invasive carcinoma. In addition, ZIC1 and GHSR promoter methylation could be detected in cervical-vaginal specimens collected by self-sampling and was found to be associated with the presence of an underlying high-grade CIN lesion or invasive cervical cancer. Even more advantageous, it was also shown that ZIC1 and GHSR promoter methylation could be detected in urine samples. Such a detection of course enables a much quicker and less cumbersome method of obtaining a sample.

Both hypermethylation of the ZIC 1 and GHSR promoter can also be detected in HPV-positive and HPV-negative vulvar cancers and their high-risk precursor lesions.

Moreover, GHSR methylation is suited to diagnose endometrial and nonHPV- induced vulvar cancer.

Accordingly, the present invention provides a method for detecting HPV-induced high-grade precancerous lesions and HPV-induced invasive cancers, nonHPV- induced endometrial and vulvar cancer, said method comprising detection of hypermethylation in the ZIC1 and/or GHSR gene in a cell whereby such hypermethylation indicates the presence of HPV-induced precursor lesions with invasive potential and HPV-induced invasive cancers and nonHPV- induced endometrial and vulvar cancer.

The test cell of the subject may comprise a cell from a sample of mucosal cells, such as cervical cells, and also other tissue such as oral cavity, oropharynx, penis, vulva, anus, rectum, endometrium, ovarium and other tissues wherein a precursor lesion or cancer associated with HPV or nonHPV- induced gynaecological, anogenital cancer or oropharyngeal cancer is to be detected. All such samples may be used as a sample in a method of the present invention. Preferably, a sample of a patient's cells comprise cervical cells or other epithelial cells of the anogenital or oropharyngeal tract as test cells. The cervical cells may e.g. be presented as a histological or cytological specimen. Cytological specimens comprise conventional cervical smears as well as thin layer preparations of cervical specimens and cervico-vaginal or vaginal specimens collected by self-sampling. Alternatively, cells may be presented in urine samples. A test cell wherein the present invention is especially advantageous over other known methods of detecting cancers in the cervix and adjacent tissues is a test cell obtained from a self-sample.

A method of the present invention is particularly suited for the detection of high-grade precancerous lesions and invasive cancers associated with ZIC1 and/or GHSR that are induced by high-risk HPVs or derived from the (female) anogenital tract. A method of detecting HPV-induced high-grade precancerous lesions with invasive potential, HPV-induced invasive cancers and nonHPV- induced endometrial cancer and vulvar cancer may comprise measuring the *ZIC1* and/or *GHSR* promoter.

Figure 1 shows the CpG-rich promoter region of the ZIC1 gene as well as the coding sequence.

Figure 2 shows the CpG-rich promoter region of the GHSR gene as well as the coding sequence.

Detection of methylation is performed on nucleic acid, such as DNA.-The reagents that are used are typically a nucleic acid (DNA) probe or (PCR) primer or a restriction endonuclease, preferably a methylation sensitive restriction endonuclease for the detection of the presence of methyl groups on the test cell DNA.

The test cell component may be detected directly *in situ* or it may be isolated from other cell components by common methods known to those of skill in the art before contacting with the reagent (see for example, "Current Protocols in Molecular Biology", Ausubel et al. 1995. 4th edition, John Wiley and Sons; "A Laboratory Guide to RNA: Isolation, analysis, and synthesis", Krieg (ed.), 1996, Wiley-Liss; "Molecular Cloning: A laboratory manual", J. Sambrook, E.F. Fritsch. 1989. 3 Vols, 2nd edition, Cold Spring Harbor Laboratory Press)

Since examples presented show frequent methylation of the ZIC 1 promoter, it is desirable to directly determine whether the *ZIC1* gene is hypermethylated. Similarly, it is also desirable to directly determine whether the *GHSR* gene is hypermethylated. In particular, the cytosine rich areas termed "CpG islands", which are primarily situated in the 5' regulatory regions of genes are normally unmethylated. The term "hypermethylation" includes any methylation of cytosine at a position that is normally unmethylated in the *ZIC1* or GHSR gene sequence (e. g. the *ZIC1* or *GHSR* promoter, first exon and first intronic sequence, see Figures 1 and 2, respectively). DNA methylation can be detected by the following assays currently used in scientific research:
- Methylation-Specific PCR (MSP), which is based on a chemical reaction of sodium bisulfite with DNA that converts unmethylated cytosines of CpG dinucleotides to uracil or UpG, followed by traditional PCR. However, methylated cytosines will not be converted in this process, and primers are designed to overlap the CpG site of interest, which allows one to determine methylation status as methylated or unmethylated.
- Whole genome bisulfite sequencing, also known as BS-Seq, which is a high-throughput genome-wide analysis of DNA methylation. It is based on aforementioned sodium bisulfite conversion of genomic DNA, which is then sequenced on a Next-generation sequencing platform. The sequences obtained are then re-aligned to the reference genome to determine methylation states of CpG dinucleotides based on mismatches resulting from the conversion of unmethylated cytosines into uracil.
- The HELP assay, which is based on restriction enzymes' differential ability to recognize and cleave methylated and unmethylated CpG DNA sites.
- ChIP-on-chip assays, which is based on the ability of commercially prepared antibodies to bind to DNA methylation-associated proteins like MeCP2.
- Restriction landmark genomic scanning, a complicated and now rarely-used assay based upon restriction enzymes' differential recognition of methylated and unmethylated CpG sites; the assay is similar in concept to the HELP assay.
- Methylated DNA immunoprecipitation (MeDIP), analogous to chromatin immunoprecipitation, immunoprecipitation is used to isolate methylated DNA fragments for input into DNA detection methods such as DNA microarrays (MeDIP-chip) or DNA sequencing (MeDIP-seq).
- Pyrosequencing of bisulfite treated DNA. This is sequencing of an amplicon made by a normal forward primer but a biatenylated reverse primer to PCR the gene of choice. The Pyrosequencer then analyses the sample by denaturing the DNA and adding one nucleotide at a time to the mix according to a sequence given by the user. If there is a mis-match, it is recorded and the percentage of DNA for which the mis-match is present is noted. This gives the user a percentage methylation per CpG island.
- Molecular break light assay for DNA adenine methyltransferase activity - an assay that relies on the specificity of the restriction enzyme DpnI for fully methylated (adenine methylation) GATC sites in an oligonucleotide labeled with a fluorophore and quencher. The adenine methyltransferase methylates the oligonucleotide making it a substrate for DpnI. Cutting of the oligonucleotide by DpnI gives rise to a fluorescence increase.
- Methyl Sensitive Southern Blotting is similar to the HELP assay, although uses Southern blotting techniques to probe gene-specific differences in methylation using restriction digests. This technique is used to evaluate local methylation near the binding site for the probe.
- Quantum-dot based methylation assay - an assay as described in Bailey, V. et al. (Genome Res. 19:1455-1461, 2009) in which the high specificity of MSP and the high sensitivity and simplicity of the quantum dot FRET (QD-FRET) technology (Zhang, C. et al., 2005, Nat. Mater. 4:826-831) is combined.
- DNA methylation detection using nanochip technology. This technique is able to detect DNA methylation at high sensitivity and specificity in minimal amounts of clinical material, without the need for bisulfite conversion and PCR amplification. Methods using solid states nanopores have been described by Shim, J. et al. (Sci. Rep. 3:1389, 2013). A device for lab on a chip technology is described in patent publication WO2009104967 (A1).

Hypermethylation preferably can be detected by restriction endonuclease treatment of the *ZIC1* or *GHSR* polynucleotide (gene) and Southern blot analysis. Any restriction endonuclease that includes CG as part of its recognition site and that is inhibited when the C is methylated, can be utilized. Methylation sensitive restriction endonucleases such as BssHII, MspI, NotI or HpaII, used alone or in combination, are examples of such endonucleases. Other methylation sensitive restriction endonucleases will be known to those of skill in the art.

An alternative preferred means to test for methylated sequences is a methylation specific PCR, which is also based on bisulfite modification of DNA, followed by specific PCR reactions that target CpG rich sequences.

A third preferred means to detect methylated sequences and to discriminate between methylated and unmethylated DNA is based on nanotechnology.

For purposes of the invention nucleic acid probe specific for ZIC 1 or GHSR may be used to detect the presence of *ZIC1* or *GHSR* polynucleotide (using nucleic acid probe) in biological fluids or tissues. Oligonucleotide primers based on any coding sequence region and regulatory sequence region in the *ZIC1* or *GHSR* sequence are useful for amplifying DNA, for example by PCR.

When using PCR primers, nucleic acid probes or restriction endonucleases, the 5' regulatory region, first intronic sequence and coding sequence of the *ZIC1* or *GHSR* sequence (as specified in Figures 1 and 2 respectively) is analysed.

Any specimen containing a detectable amount of *ZIC1* or *GHSR* polynucleotide can be used. Preferred samples for testing according to methods of the invention include such specimens as (cervical or vaginal) scrapes, cervico-vaginal lavages or swabs, urine, blood and/or (cervical) biopsies and the like. Although the subject can be any mammal, preferably the subject is human.

Diagnostic methods for the detection of disorders include methods wherein a sample for testing is provided, which sample comprises a cell preparation from cervical or other tissue. Preferably such samples are provided as smears or other cytological samples. Additional suitable samples include urine and blood.

That self-samples can be used to detect changes in promoter methylation in markers that reliably predict a risk for or the presence of HPV-induced high-grade precancerous lesions and HPV-induced invasive cancers and nonHPV-induced gynaecological and anogenital cancers is not obvious if the same detection can be achieved in doctor-obtained samples.

Self-samples and physician taken cervical scrapes have a different cellular composition, reflected by a different fraction of indicator cells exfoliated from cervical lesions over normal cells, which are mostly vaginal cells in case of self-samples. Hence in self-samples a more random distribution of vaginal and cervical indicator cells is seen. The mostly lower proportion of hypermethylation-positive cervical indicator cells in self-samples is known to affect the performance of cytomorphological and molecular tests on these samples. This is supported by literature data showing that cytology (cytomorphological analysis to detect abnormal cells) cannot be reliably performed on self-sampled material (Nobbenhuis et al, J. Clin. Pahtol. 55:435, 2002; Garcia et al, Obstet Gynecol 102: 266-272, 2003).

Moreover, studies that describe a direct comparison between self-samples and physician taken scrapes for the presence of hypermethylated indicator cells, by assessment of DNA methylation levels of host cell genes, show a lower sensitivity for the detection of CIN 3 in self-samples compared to cervical scrapes:
- Chang, BMC Cancer 15:418, 2015 who shows in Figure 2 that less CIN 3 are detected by methylation analysis on self-samples than on scrapes;
- Luttmer et al, Br J Cancer 2016 , Jul 14. doi: 10.1038/bjc.2016.200 in which it is shown that CIN3 sensitivity of FAM19A4 methylation analysis was 78.4% in self-samples and 88.2% in scrapes;
- Boers et al., Br J Cancer 111: 1095-1101, 2015 who show that CIN3+ sensitivity of JAM3 is 71% in self-samples and 82% in scrapes)
Moreover, the overall methylation levels/ratios are often lower in self-samples compared to corresponding physician taken scrapes (Most clearly shown for hTERT in Figure 3 by Boers et al.,).

In conclusion, 1) the unreliability of cytological analysis on self-samples to detect CIN3, 2) the lower sensitivity of methylation analysis for the detection of CIN using self-samples compared to physician taken scrapes, and 3) the lower methylation levels found in self-samples compared to physician taken scrapes, are all indicative of a different cellular composition and lower fraction of indicator cells in self samples compared to physician taken scrapes.

It is thus surprising that hypermethylation of the ZIC 1 and/or GSHR promoter, when assessed on self-samples provides a reliable measure for the detection of HPV-induced high-grade precancerous lesions and HPV-induced invasive cancers and nonHPV-induced gynaecological and anogenital cancers. Next to their high specificity they also enable the detection of all (100%) cervical carcinomas.

Further, the sample may be derived from a urine sample. As is shown in Example 8, it has been shown feasible to detect hypermethylation in a urine sample. This would of course be very advantageous in those cases where it is difficult to obtain cervical samples.

A cell or tissue sample obtained from a mammal, preferably a human, is suitably pre-treated to allow contact between the cellular DNA of a test cell comprised in said sample with a reagent that detects ZIC1 or GHSR and detects an alteration in the methylation of the ZIC1 or GHSR gene as compared to that of a comparable normal cell. Samples may be mounted on a suitable support to allow observation of individual cells. Examples of well-known support materials include glass, polystyrene, polypropylene, polyethylene, polycarbonate, polyurethane, optionally provided with layers to improve cell adhesion and immobilization of the sample, such as layers of poly-L-lysine or silane. Cervical smears or biopsies may for instance be prepared as for the Papanicolaou (Pap) test or any suitable modification thereof as known by the skilled person, and may be fixed by procedures that allow proper access of the reagent to the target component. In certain embodiments of the invention the cytological specimens are provided as conventional smear samples or thin layer preparations of cervical cells or liquid based cytology samples or any other kind of preparation known to those of skill in the art. If storage is required, routine procedures use buffered formalin for fixation followed by paraffin embedding, which provides for a well-preserved tissue infrastructure.

In one embodiment of a method of the invention an increased methylation of the *ZIC1* and/or *GHSR* promoter in the test cell is detected as compared to the comparable normal cell.

The present invention also provides a kit of parts as defined in the claims, for use in a method of detecting HPV-induced precursor lesions with invasive potential, HPV-induced invasive cancers and nonHPV- induced gynaecological and anogenital cancers associated with ZIC 1 and/or GHSR in test cells of a subject. Such a kit may suitably comprise a brush or spatula to take a (cervical) scrape together with a container filled with collection medium to collect test cells. Alternatively, a sampling device consisting of an irrigation syringe, a disposable female urine catheter and a container with irrigation fluid will be included to collect cervical cells by cervico-vaginal lavage. Additionally, a container to collect urine is suitable, preferably to be used to collect first-void urine. A kit according to the present invention may comprise primers and probes for the detection of ZIC1 and/or GHSR promoter methylation-.

A kit of parts according to the invention comprises means for the detection of *ZIC1* and/or *GHSR* promoter methylation, such as, methylationsensitive restriction enzymes, or probes or primers capable of hybridising to the nucleotide sequence of Fig. 1 and/or Fig. 2.

In yet another alternative embodiment of a kit of the invention the means for the detection of *ZIC1* and/or *GHSR* promoter methylation may be combined with means for the detection of HPV infection, preferably for the detection of HPV infection of the high-risk type. Such means may comprise HPV-specific primers or probes, protein markers for HPV infection or even surrogate markers for HPV infection as are known in the art.

The present invention will now be illustrated by way of the following, non limiting examples.

### EXAMPLES

### Example 1. Discovery of ZIC1 and GHSR as methylation targets in HPV-induced transformation

A comprehensive analysis of genome-wide DNA methylation changes associated with HPV-induced transformation in vitro and cervical carcinogenesis in vivo has been conducted by means of MBD-sequencing of consecutive passages of HPV16 and HPV18-immortalized keratinocytes as well as cervical tissue specimens. Cell cultures included were 2 DNA isolates of primary human foreskin keratinocytes, 10 DNA isolates of keratinocytes transfected with full length HPV16 and HPV18 DNA (different passages of cell lines FK16A, FK16B, FK18A, FK18B; Steenbergen et al., Oncogene 1996, 13(6):1249-57), 2 DNA isolates of keratinocytes transduced with HPV16E6E7 (Steenbergen et al., J. Pathology 2013; 231(1):53-62) and the cervical cancer cell line SiHa. The cervical tissue specimens included consisted of 10 carcinomas, 12 high-grade cervical intraepithelial neoplasia(CIN2/3) and 3 low-grade cervical intraepithelial neoplasia(CIN1).

The MBD-sequencing method is based on the enrichment of CpG methylated fragments using methyl binding domains followed by massive parallel sequencing (Serre et al., Nucleic Acids Res. 2010;38(2):391-9). By mapping these fragments to a reference genome, the putatively methylated locus can be determined.

By this approach we identified ZIC1 and GHSR as novel methylation targets in HPV-induced transformation in vitro and cervical carcinogenesis in vivo.

Methylation of the ZIC1 and GHSR promoter regions was verified by quantitative Methylation Specific PCR (qMSP) in primary keratinocytes, early and late passages of HPV16 and HPV18-immortalized keratinocytes and cervical cancer cell lines. Primers and probes for qMSP are listed in Table 1. The housekeeping gene β-actin (ACTB) was chosen as a reference for total DNA input measurement. Quantification was performed using the comparative Ct method (Schmittgen et al., Nat Protoc 2008, 3:1101-1108). The levels of methylation of ZIC1 and GHSR increase with stage of transformation and are significantly increased in late passage HPV16 and HPV18 immortalized keratinocytes and cervical cancer cells compared to primary keratinocytes and early passage HPV16 and HPV18 immortalized keratinocytes (Figure 3).

### Example 2: ZIC1 and GHSR promoter methylation are common events in high grade CIN lesions, cervical squamous cell carcinomas, adenosquamous carcinomas, adenocarcinomas and neuroendocrine carcinomas

Next, we analysed ZIC1 and GHSR promoter methylation in cervical tissue specimens by qMSP. Tissue samples tested included 30 normal cervical control samples, 13 CIN1 lesions, 29 CIN3 lesions and 24 cervical squamous cell carcinomas (SCC) . The housekeeping gene β-actin (ACTB) was chosen as a reference for total DNA input measurement. Quantification was performed using the comparative Ct method (Schmittgen et al., Nat Protoc 2008, 3:1101-1108). We found that ZIC1 promoter methylation was significantly increased in CIN3 lesions and SCC compared to normal and CIN1 lesions. Moreover methylation levels were significantly increased in SCC compared to CIN3 lesions (Figure 4A). At a threshold setting resulting in 90% specificity, nearly all (96%) SCC and 79% of CIN3 lesions were positive for ZIC1 methylation. Next to cervical squamous cell carcinomas we also analysed ZIC1 promoter methylation in cervical adenocarcinomas. Adenocarcinomas, which constitute up to 20% of cervical carcinomas, are of particular interest as the incidence of cervical adenocarcinoma has remained the same or even increased in countries with a nation-wide cervical screening programme. This indicates that cervical adenocarcinoma and its glandular precursor lesion, i.e. adenocarcinoma in situ (ACIS), are frequently missed by cytology based screening. Based on comparative genetic and epigenetic studies between cervical squamous cell carcinomas and cervical adenocarcinomas it has been found that both tumor histotypes develop via distinct carcinogenenic pathways (Dong et al., 2001, Kang et al., 2005, Wilting et al., 2006, Henken et al., 2007). Consequently, most biomarkers enabling the detection of cervical squamous cell carcinoma do not necessarily detect cervical adenocarcinoma.

Interestingly, ZIC1 promoter methylation appeared to be an exception as, in contrast to most known markers, as ZIC1 methylation levels were increased in all but one (86%) AdCa tested (Figure 4A). Similar results have been obtained for cervical adenosquamous carcinomas and neuroendocrine carcinomas.

Therefore, ZIC 1 promoter methylation appears to be a universal methylation marker for all cervical carcinoma histotypes.

Analysis of GHSR promoter methylation revealed significantly increased methylation levels increased in CIN3 lesions, SCC and AdCa compared to normal and CIN1 lesions. Moreover methylation levels were significantly increased in SCC compared to CIN3 lesions (Figure 4B). At a threshold setting resulting in 90% specificity, all (100%) SCC, all (100%) AdCa and 69% of CIN3 lesions were positive for GHSR methylation. Hence, GHSR promoter methylation also represents a universal methylation marker for all cervical carcinoma histotypes

### Example 3. Detection of ZIC1 and GHSR promoter methylation in hrHPV-positive cervical scrapes

From women participating in a population-based screening we studied cervical scrapes of hrHPV positive women in which CIN3 (n=56) was diagnosed, and hrHPV negative and positive women in whom at maximum CIN 1 was diagnosed (n=40 and n=87, respectively). Additionally, cervical scrapes of women diagnosed with SCC (n=23) and AdCa (n=3) of the cervix were tested. Cervical scrapes of these women were collected in preservation medium in which nucleic acids are well preserved.

ZIC1 methylation was significantly increased in scrapes of women with CIN3, SCC and AdCa compared to HPV negative and HPV positive controls. Methylation levels were also significantly increased in scrapes of women with SCC compared to women with CIN3 (Figure 5A). At a threshold setting resulting in 90% specificity, all (100%) scrapes of women SCC and AdCa were positive, and 56% of CIN3 lesions tested ZIC1 methylation positive. At 70% specificity, 86% of CIN3 lesions are detected.

GHSR methylation was significantly increased in scrapes of women with CIN3, SCC and AdCa compared to HPV negative and HPV positive controls. Methylation levels were also significantly increased in scrapes of women with SCC compared to women with CIN3 (Figure 5B). At a threshold setting resulting in 90% specificity, all (100%) scrapes of women SCC and AdCa were positive, and 58% of CIN3 lesions tested positive for GHSR methylation. At 70% specificity, 80% of CIN3 lesions are detected. Current data indicate that both ZIC1 and GHSR methylation analysis enable the detection of underlying cervical disease (CIN3+) in cervical scrapes at a high sensitivity and specificity. Particularly at high specificity settings both ZIC 1 and GHSR outperform other published methylation markers and detect all cancers.

Thereby these genes provide promising triage markers in screening by primary HPV testing.

### Example 4: ZIC1 and GHSR promoter methylation as marker for primary screening

Methylation markers tested so far are not well suitable for use in primary screening due to a too low specificity at an acceptable sensitivity for CIN2/3 and cancer. For example markers or marker panels consisting of various combination of CADM1, MAL, miR-124 and/or FAM19A4 have a specificity of 70-75% at acceptable sensitivity of CIN2/3 (70%) and up to 100% detection of cervical cancer.

When evaluating the use of ZIC1 and GHSR methylation as marker for primary screening it was surprisingly found that both these markers have an extremely high sensitivity for detecting cancer at an extremely high specificity. At 100% specificity based on HPV-negative controls (and 90% specificity based on HPV-positive controls) ZIC1 methylation detects 100% of SCC, 100% of AdCa and 55% of CIN3 lesions.

At 95% specificity based on HPV-negative controls (and 90% specificity based on HPV-positive controls) GHSR methylation detects 100% of SCC, 100% of AdCa and 57% of CIN3 lesions. Hence primary screening by ZIC1 and/or GHSR enables the detection of virtually all cervical carcinomas at very high specificity (95-100%).

### Example 5: ZIC1 and GHSR promoter methylation in self-sampled specimens

We subsequently analysed self-sampled cervico-vaginal specimens collected using either a VibaBrush (Rovers Medical Devices, Oss, the Netherlands) or a Delphi-screener (Delphi BioScience BV, Scherpenzeel, The Netherlands). Self-collected samples are equivalent to physician-taken ones with respect to HPV testing (Brink et al. J Clin Microbiol 2006;44:2518-23). Testing for hrHPV in self-samples yields at least as much ≥CIN 2 lesions in this population as found by regular screening in a matched population of responder women (Bais et al., Int J Cancer: 2007, 120:1505-1510; Gök et al., BMJ 2010;340:c1040). However, also for HPV self-sampling there is a need for triage tools to stratify hrHPV positive women into those with and without ≥CIN 2/3 and ≥adenocarcinoma in situ. Whereas conventional cytology cannot be reliably performed on self-collected cervico-vaginal specimens (Brink et al. J Clin Microbiol 2006;44:2518-23), DNA methylation analysis can be applied to self-collected cervico-vaginal lavages (Eijsink et al., Gynecol Oncol 2011;120:280-3.). Importantly, the present findings show that the methylation markers GHSR and ZIC1 enable the detection of underlying CIN2+ not only when applied to self-collected cervico-vaginal lavage specimens but self-collected vaginal brush samples as well. The latter is a specimen type in which previous known markers often performed with low clinical sensitivity. Consequently, the ZIC1 and GHSR markers can be considered as pan-detection markers performing equal on physician-taken cervical smears, lavage-based self-samples and brush-based self-samples.

A series of 20 self-collected vaginal brush samples of hrHPV positive women without evidence of clinically meaningful disease in follow-up as well as 25 self-collected vaginal brush samples of hrHPV positive women with an abnormal follow-up smear and an underlying lesion ≥ CIN3 were tested by qMSP for ZIC1 and GHSR promoter methylation. Virtually all of self-collected vaginal brush samples of women that later were diagnosed with cancer tested positive of ZIC1 and GHSR methylation at a specificity of 70%. Additionally, more than half of self-collected vaginal brush samples of women that later were diagnosed with CIN3 tested positive for ZIC1 and GHSR promoter methylation.

Most interestingly, analysis of 24 self-collected cervico-vaginal lavage specimens of hrHPV positive women, revealed a detection of 100% of carcinomas at a specificity of up to 100% using ZIC1 or GHSR methylation analysis. ZIC1 methylation even detected 70% of CIN3 lesions at 90% specificity. Compared to known methylation markers applied to self-collected cervico-vaginal lavage specimens (earlier cited literature of Chang, Boers et al. and Luttmer et al., Eijsink et al., Gynecol Oncol 2011;120:280-3; Verhoef et al., Lancet Oncol 2014; 15: 315-22) the marker potential of ZIC1 in self-collected cervico-vaginal lavage specimens is remarkably high.

These data show that ZIC1 and GHSR promoter methylation analysis on self-sampled materials obtained from both self-collected vaginal brush samples and self-collected cervico-vaginal lavage specimens is well feasible and will improve the detection of underlying high-grade cervical disease. Most interestingly ZIC1 methylation analysis on self-collected cervico-vaginal lavage specimens is even suitable for primary screening (100% sensitivity for cervical cancer at 100% specificity)

### Example 6: GHSR promoter methylation in cervical scrapes of women with endometrial carcinoma

A total of 24 cervical scrapes of women with endometrial carcinoma were tested for GHSR methylation. Compared to cervical scrapes of hrHPV negative and positive women in whom at maximum CIN 1 was diagnosed (n=40 and n=87, respectively), GHSR methylation levels were significantly increased in women with endometrial carcinoma (Figure 6). At specific threshold settings at which ~8% of controls tested positive, 80% of endometrial carcinomas were detected.

### Example 7: ZIC1 and GHSR promoter methylation in HPV-positive and HPV-negative vulvar cancers and their high-grade precursor lesions.

Like cervical precancerous lesions most vulvar precancerous lesions (vulvar intraepithelial neoplasia; VIN) result from an infection with HPV. Only a small subset of the lesions is at increased risk of progression to vulvar squamous cell carcinoma (VSCC). To determine whether ZIC1 and GHSR methylation can distinguish the lesion with a high risk of progression to cancer from those with a low cancer progression risk, the following tissue specimens were analysed by qMSP:
Normal vulva tissues (n=8; control vulvar tissues obtained from labiaplasty), HPV-positive VIN lesions of women that did not develop VSCC during at least 10 year follow-up (n=15; lesions with low risk of progression to cancer), HPV-positive VIN lesions of women with VSCC (n=15; lesions with high risk of progression to cancer) and vulvar squamous cell carcinomas (VSCC; n=50; including 38 HPV-negative and 12 HPV-positive cancers).

For both ZIC1 and GHSR methylation levels were significantly increased in HPV-positive VIN lesions of women with VSCC compared to HPV-positive VIN of women without VSCC. Moreover, methylation levels in VIN lesions associated with VSCC were similar to methylation levels detected in VSCC. This indicates that ZIC1 and GHSR methylation can be used for risk assessment of HPV-induced precancerous lesions of the vulva.

### Example 8: ZIC1 and GHSR promoter methylation in urine samples of cervical cancer patients

Given the fact that HPV testing in urine has shown a promising alternative to HPV testing on cervical scrapes (Pathak et al., BMJ 2014;349:g5264), we evaluated whether methylation marker detection in urine enables the detection of cervical cancer.

Hereto we analysed ZIC1 and GHSR methylation in urine samples collected from 16 cervical cancer patients and in 20 urine samples of age-matched female controls.

As shown in Figure 7 it was found that both GHSR and ZIC1 methylation levels are significantly increased in cervical cancer patients compared to controls (p<0.01). Receiver operating curve (ROC) analysis yielded an area under the curve (AUC) of 0,994 for ZIC1 and 1,0 for GHSR. This indicates that ZIC1 and GHSR methylation analysis on urine is suitable for primary screening.

**Table 1 Primer and probe sequences (5'-3') used for ZIC1 and GHSR quantitative MSP analysis**

| | **Forward primer** | **Reverse primer** | **Probe** |
|---|---|---|---|
| ZIC1 | GGGCGGGTTAATGAGTTGC | TCACGTACTACCGACGCTAACG | CGCCGCGCCAACGAAAAAC |
| GHSR | GTTTGGTTTTTGCGGTTTTTATTC | CAACCCTACCTCGCATTTACG | CTCGATCCAATTCCATCTCGC ACTTCC |

## Claims

1. A method for detecting HPV-induced high-grade precancerous lesions and HPV-induced invasive cancers and nonHPV-induced endometrial cancer and vulvar cancer, said method comprising:
- detection of hypermethylation in the GHSR and/or ZIC1 gene in a cell whereby such hypermethylation indicates the presence of HPV-induced high-grade precancerous lesions or HPV-induced invasive cancers,
- detection of hypermethylation in the GHSR and ZIC1 gene in a cell whereby such hypermethylation indicates the presence of nonHPV-induced endometrial cancer, or nonHPV-induced vulvar cancer, or
- detection of hypermethylation in the ZIC1 gene in a cell whereby such hypermethylation indicates the presence of nonHPV-induced endometrial cancer or vulvar cancer.

2. Method according to claim 1, wherein said HPV-induced high-grade precancerous lesion or HPV-induced invasive carcinoma is a high-grade premalignant cervical lesion or invasive cervical cancer.

3. Method according to claim 1 or 2, wherein said HPV-induced invasive cancer is a high-risk HPV-induced invasive cancer.

4. Method according to claim 1, wherein said nonHPV-induced gynaecological cancers is an endometrial cancer.

5. Method according to any one of the preceding claims, wherein said hypermethylation is detected in the CpG rich sequences as indicated in Figures 1 and 2.

6. A method of detecting HPV-induced high-grade precancerous lesion and HPV-induced invasive cancers and nonHPV-induced endometrial cancer and vulvar cancer according to any of claims 1-5, wherein
- said hypermethylation in the GHSR and/or ZIC1 gene that indicates the presence of HPV-induced high-grade precancerous lesions is an increased methylation of GHSR and/or ZIC1 CpG rich promoter and/or gene sequences in the test cell as compared to the comparable normal cell; or
- said hypermethylation in the GHSR and ZIC1 gene that indicates the presence of nonHPV-induced endometrial cancer, and nonHPV induced vulvar cancer, is an increased methylation of GHSR CpG rich promoter and/or gene sequence and ZIC1 CpG rich promoter and/or gene sequences in the test cell as compared to the comparable normal cell; or
- said hypermethylation in the ZIC1 gene that indicates the presence of nonHPV-induced endometrial cancer or vulvar cancer is an increased methylation of ZIC1 CpG rich promoter and/or gene sequences in the test cell as compared to the comparable normal cell.

7. Method according to any of claims 1-6, wherein the reagent is a methylation sensitive restriction endonuclease, chosen from the group consisting of BssHII, MspI, NotI and HpaII.

8. Method according to any of claims 1-6 wherein the method involves nanotechnology, preferably lab on a chip technology.

9. Method according to claim any of claims 1-6, wherein a methylation specific PCR is performed, which is based on bisulfite modification of DNA, followed by specific PCR reactions that target CpG rich sequences, preferably wherein the reagent is a nucleic acid probe or primer that binds to the nucleic acid as indicated in Figure 1 or 2, more preferably, wherein said nucleic acid probe or primer has a detectable label.

10. Method according to claim 7, 8 or 9, wherein the nucleic acid probe has a nucleotide sequence selected from the group consisting of:
a) a polynucleotide sequence capable of hybridizing under stringent conditions to the sequence ZIC1 as set forth in Figure 1 or to the sequence GHSR as set forth in Figure 2;
b) a polynucleotide having at least 70% identity to one of the polynucleotides of a);
c) a polynucleotide complementary to one of the polynucleotides of a); and
d) a polynucleotide comprising at least 15 bases of one of the polynucleotides of a) or b).

11. Method according to any of claims 1 -10, wherein the methylation of both the GHSR and the ZIC1 gene is determined.

12. Use of the promoter regions of GHSR and/or ZIC1 as a molecular diagnostic marker for the detection of HPV-induced high-grade precancerous lesion or HPV-induced invasive carcinoma, wherein the methylation of said marker is predictive for the occurrence of said lesion, carcinoma or cancer.

13. Method according to any of claims 1 - 11, wherein the cell is derived from a urine sample or a cervical sample.

14. Method according to claim 13, wherein the cervical sample is a self-sample.

15. Kit of parts for use in a method of detecting HPV-induced high- grade precancerous lesion or HPV-induced invasive carcinoma or nonHPV-induced gynaecological or anogenital cancer, said kit comprising
- means for the detection of GHSR and ZIC1 methylation wherein said means comprise probes and/or primers specific for a bisulfite modified sequence of the promoter of ZIC1 nucleotide sequence as present in SEQ ID NO: 7 and a bisulfite modified sequence of the promoter of the GHSR nucleotide sequence as present in SEQ ID NO: 8;
- means for the detection of HPV infection, wherein said means comprise probes and primers specific for HPV.

## Patentansprüche

1. Verfahren zum Nachweisen von HPV-induzierten hochgradigen präkanzerösen Läsionen und HPV-induzierten invasiven Krebsen und nicht-HPV-induziertem Endometriumkrebs und Vulvakrebs, das Verfahren umfassend:
- Nachweis von Hypermethylierung in dem GHSR- und/oder ZIC1-Gen in einer Zelle, wobei solche Hypermethylierung das Vorhandensein von HPV-induzierten hochgradigen präkanzerösen Läsionen oder HPV-induzierten invasiven Krebsen anzeigt,
- Nachweis von Hypermethylierung in dem GHSR- und ZIC1-Gen in einer Zelle, wobei solche Hypermethylierung das Vorhandensein von nicht-HPV-induziertem Endometriumkrebs oder nicht-HPV-induziertem Vulvakrebs anzeigt, oder
- Nachweis von Hypermethylierung in dem ZIC1-Gen in einer Zelle, wobei solche Hypermethylierung das Vorhandensein von nicht-HPV-induziertem Endometrium- oder Vulvakrebs anzeigt.

2. Verfahren nach Anspruch 1, wobei die HPV-induzierte hochgradige präkanzeröse Läsion oder das HPV-induzierte invasive Karzinom eine hochgradige prämaligne zervikale Läsion oder ein invasiver zervikaler Krebs ist.

3. Verfahren nach Anspruch 1 oder 2, wobei der HPV-induzierte invasive Krebs ein Hochrisiko-HPV-induzierter invasiver Krebs ist.

4. Verfahren nach Anspruch 1, wobei der nicht-HPV-induzierte gynäkologische Krebs ein Endometriumkrebs ist.

5. Verfahren nach einem der vorhergehenden Ansprüche, wobei die Hypermethylierung in den CpG-reichen Sequenzen, wie in den Figuren 1 und 2 angezeigt, nachgewiesen wird.

6. Verfahren zum Nachweis von HPV-induzierten hochgradigen präkanzerösen Läsionen und HPV-induzierten invasiven Krebsen und nicht-HPV-induziertem Endometriumkrebs und Vulvakrebs nach einem der Ansprüche 1 - 5, wobei
- die Hypermethylierung in dem GHSR- und/oder ZIC1-Gen, die das Vorhandensein von HPV-induzierten hochgradigen präkanzerösen Läsionen anzeigt, eine erhöhte Methylierung von GHSR- und/oder ZIC1-CpG-reichen Promotor- und/oder Gensequenzen in der Testzelle im Vergleich zu der vergleichbaren normalen Zelle ist; oder
- die Hypermethylierung in dem GHSR- und ZIC1-Gen, die das Vorhandensein von nicht-HPV-induziertem Endometriumkrebs und nicht-HPV-induziertem Vulvakrebs anzeigt, eine erhöhte Methylierung von GHSR-CpG-reichen Promotor- und/oder Gensequenz und ZIC1-CpG-reichen Promotor- und/oder Gensequenzen in der Testzelle im Vergleich zu der vergleichbaren normalen Zelle ist; oder
- die Hypermethylierung in dem ZIC1-Gen, die das Vorhandensein von nicht-HPV-induziertem Endometriumkrebs oder Vulvakrebs anzeigt, eine erhöhte Methylierung von ZIC1-CpG-reichen Promotor- und/oder Gensequenzen in der Testzelle im Vergleich zu der vergleichbaren normalen Zelle ist.

7. Verfahren nach einem der Ansprüche 1 - 6, wobei das Reagenz eine methylierungsempfindliche Restriktionsendonuklease ist, ausgewählt aus der Gruppe bestehend aus BssHII, MspI, NotI und HpaII.

8. Verfahren nach einem der Ansprüche 1 - 6, wobei das Verfahren Nanotechnologie, vorzugsweise Lab-on-a-Chip-Technologie, involviert.

9. Verfahren nach einem der Ansprüche 1 - 6, wobei eine methylierungsspezifische PCR durchgeführt wird, die auf Bisulfit-Modifikation von DNA basiert, gefolgt von spezifischen PCR-Reaktionen, die auf CpG-reiche Sequenzen zielen, vorzugsweise wobei das Reagenz eine Nukleinsäuresonde oder Primer ist, die/der an die Nukleinsäure bindet, wie in Figur 1 oder 2 angezeigt, wobei die Nukleinsäuresonde oder der Primer bevorzugter eine nachweisbare Markierung hat.

10. Verfahren nach Anspruch 7, 8 oder 9, wobei die Nukleinsäuresonde eine Nukleotidsequenz hat, ausgewählt aus der Gruppe bestehend aus:
a) einer Polynukleotidsequenz, in der Lage, unter stringenten Bedingungen mit der Sequenz ZIC1, wie in Figur 1 dargestellt, oder mit der Sequenz GHSR, wie in Figur 2 dargestellt, zu hybridisieren;
b) einem Polynukleotid mit wenigstens 70 % Identität mit einem der Polynukleotide von a);
c) einem Polynukleotid, komplementär zu einem der Polynukleotide aus a); und
d) einem Polynukleotid, umfassend wenigstens 15 Basen eines der Polynukleotide von a) oder b).

11. Verfahren nach einem der Ansprüche 1 - 10, wobei die Methylierung sowohl des GHSR- als auch des ZIC1-Gens bestimmt wird.

12. Verwendung der Promotorregionen von GHSR und/oder ZIC1 als ein molekularer diagnostischer Marker für den Nachweis von HPV-induzierten hochgradigen präkanzerösen Läsion oder eines HPV-induzierten invasiven Karzinoms, wobei die Methylierung des Markers prädiktiv für das Auftreten der Läsion, des Karzinoms oder des Krebses ist.

13. Verfahren nach einem der Ansprüche 1 - 11, wobei die Zelle aus einer Urinprobe oder einer Zervixprobe stammt.

14. Verfahren nach Anspruch 13, wobei die Zervixprobe eine Selbstprobe ist.

15. Teilesatz zur Verwendung in einem Verfahren zum Nachweis einer HPV-induzierten hochgradigen präkanzerösen Läsion oder eines HPV-induzierten invasiven Karzinoms oder eines nicht-HPV-induzierten gynäkologischen oder anogenitalen Krebses, der Satz umfassend
- Mittel für den Nachweis von GHSR und ZIC1-Methylierung, wobei die Mittel Sonden und/oder Primer umfassen, spezifisch für eine Bisulfit-modifizierte Sequenz des Promotors von ZIC1-Nukleotidsequenz, wie sie in SEQ ID NR: 7 vorliegt und eine Bisulfit-modifizierte Sequenz des Promotors von GHSR-Nukleotidsequenz, wie sie in SEQ ID NR: 8 vorliegt;
- Mittel für den Nachweis von HPV-Infektion, wobei diese Mittel HPV-spezifische Sonden und Primer umfassen.

## Revendications

1. Procédé de détection de lésions précancéreuses de haut grade induites par HPV et de cancers invasifs induits par HPV et de cancers de l'endomètre et de cancers de la vulve non induits par HPV, ledit procédé comprenant les étapes consistant à :
- détecter une hyperméthylation dans le gène GHSR et/ou ZIC1 dans une cellule, cette hyperméthylation indiquant la présence de lésions précancéreuses de haut grade induites par HPV ou de cancers invasifs induits par HPV,
- détecter une hyperméthylation dans le gène GHSR et ZIC1 dans une cellule, cette hyperméthylation indiquant la présence d'un cancer de l'endomètre non induit par HPV ou d'un cancer de la vulve non induit par HPV, ou
- détecter une hyperméthylation dans le gène ZIC1 dans une cellule, cette hyperméthylation indiquant la présence d'un cancer de l'endomètre ou d'un cancer de la vulve non induit par HPV.

2. Procédé selon la revendication 1, dans lequel ladite lésion précancéreuse de haut grade induite par HPV ou ledit carcinome invasif induit par HPV est une lésion cervicale prémaligne de haut grade ou un cancer invasif du col de l'utérus.

3. Procédé selon la revendication 1 ou 2, dans lequel ledit cancer invasif induit par HPV est un cancer invasif induit par HPV à haut risque.

4. Procédé selon la revendication 1, dans lequel lesdits cancers gynécologiques non induits par HPV sont un cancer de l'endomètre.

5. Procédé selon l'une quelconque des revendications précédentes, dans lequel ladite hyperméthylation est détectée dans les séquences riches en CpG, comme indiqué dans les figures 1 et 2.

6. Procédé de détection de lésions précancéreuses de haut grade induites par HPV et de cancers invasifs induits par HPV et de cancers de l'endomètre et de cancers de la vulve non induits par HPV selon l'une quelconque des revendications 1 à 5, dans lequel
- ladite hyperméthylation dans le gène GHSR et/ou ZIC1 qui indique la présence de lésions précancéreuses de haut grade induites par HPV est une méthylation accrue de promoteur et/ou de séquences géniques riche(s) en CpG GHSR et/ou ZIC1 dans la cellule de test par rapport à la cellule normale comparable ; ou
- ladite hyperméthylation dans le gène GHSR et ZIC1 qui indique la présence d'un cancer de l'endomètre non induit par HPV, et d'un cancer de la vulve non induit par HPV, est une méthylation accrue du promoteur et/ou de la séquence génique riche en CpG GHSR et du promoteur et/ou de séquences géniques riche(s) en CpG ZIC1 dans la cellule de test par rapport à la cellule normale comparable ; ou
- ladite hyperméthylation dans le gène ZIC1 qui indique la présence d'un cancer de l'endomètre ou d'un cancer de la vulve non induit par HPV est une méthylation accrue de promoteur et/ou de séquences géniques riche(s) en CpG ZIC1 dans la cellule de test par comparaison à la cellule normale comparable.

7. Procédé selon l'une quelconque des revendications 1 à 6, dans lequel le réactif est une endonucléase de restriction sensible à une méthylation, choisie dans le groupe constitué de BssHII, Mspl, Notl et HpaII.

8. Procédé selon l'une quelconque des revendications 1 à 6, dans lequel le procédé implique une nanotechnologie, de préférence une technologie de type laboratoire sur puce.

9. Procédé selon l'une quelconque des revendications 1 à 6, dans lequel une PCR spécifique à une méthylation est effectuée, qui est basée sur une modification au bisulfite d'ADN, suivie de réactions de PCR spécifiques qui ciblent des séquences riches en CpG, de préférence dans lequel le réactif est une sonde ou une amorce d'acide nucléique qui se lie à l'acide nucléique comme indiqué dans la figure 1 ou 2, de préférence dans lequel ladite sonde ou amorce d'acide nucléique a un marqueur détectable.

10. Procédé selon la revendication 7, 8 ou 9, dans lequel la sonde d'acide nucléique présente une séquence nucléotidique choisie dans le groupe consistant en :
a) une séquence polynucléotidique capable de s'hybrider dans des conditions strictes à la séquence ZIC1 telle que définie à la figure 1 ou à la séquence GHSR telle que définie à la figure 2 ;
b) un polynucléotide présentant au moins 70 % d'identité avec l'un des polynucléotides de a) ;
c) un polynucléotide complémentaire de l'un des polynucléotides de a) ; et
d) un polynucléotide comprenant au moins 15 bases d'un des polynucléotides de a) ou b).

11. Procédé selon l'une quelconque des revendications 1 à 10, dans lequel la méthylation à la fois du gène GHSR et du gène ZIC1 est déterminée.

12. Utilisation des régions de promoteur de GHSR et/ou ZIC1 en tant que marqueur de diagnostic moléculaire pour une détection d'une lésion précancéreuse de haut grade induite par HPV ou d'un carcinome invasif induit par HPV, dans laquelle la méthylation dudit marqueur est prédictive de l'apparition de ladite lésion, dudit carcinome ou dudit cancer.

13. Procédé selon l'une quelconque des revendications 1 à 11, dans lequel la cellule est dérivée d'un échantillon d'urine ou d'un échantillon cervical.

14. Procédé selon la revendication 13, dans lequel l'échantillon cervical est un auto-échantillon.

15. Kit de pièces à utiliser dans un procédé de détection d'une lésion précancéreuse de haut grade induite par HPV ou d'un carcinome invasif induit par HPV ou d'un cancer gynécologique ou anogénital non induit par HPV, ledit kit comprenant
- des moyens de détection de méthylation de GHSR et de ZIC1, dans lequel lesdits moyens comprennent des sondes et/ou des amorces spécifiques d'une séquence modifiée au bisulfite du promoteur de la séquence nucléotidique ZIC1 telle que présente dans SEQ ID NO : 7 et une séquence modifiée par bisulfite du promoteur de la séquence nucléotidique de GHSR telle que présente dans SEQ ID NO : 8 ;
- des moyens de détection d'infection par HPV, lesdits moyens comprenant des sondes et des amorces spécifiques pour HPV.
